**Europäisches Patentamt**

**European Patent Office**

**Office européen des brevets**

(19)

(11) Veröffentlichungsnummer : **0 040 232 B2**

(12) # NEUE EUROPÄISCHE PATENTSCHRIFT

(45) Veröffentlichungstag der neuen Patentschrift : **29.09.93 Patentblatt 93/39**

(51) Int. Cl.$^5$ : **A61K 6/06, C09C 1/30**

(21) Anmeldenummer : **80902285.8**

(22) Anmeldetag : **21.11.80**

(86) Internationale Anmeldenummer : **PCT/EP80/00135**

(87) Internationale Veröffentlichungsnummer : **WO 81/01366 28.05.81 Gazette 81/13**

(54) **VERWENDUNG VON KIESELSÄUREGRANULATEN ALS FÜLLSTOFFE FÜR DENTALMASSEN.**

(30) Priorität : **22.11.79 DE 2947129**

(43) Veröffentlichungstag der Anmeldung : **25.11.81 Patentblatt 81/47**

(45) Bekanntmachung des Hinweises auf die Patenterteilung : **02.11.83 Patentblatt 83/44**

(45) Bekanntmachung des Hinweises auf die Entscheidung über den Einspruch : **29.09.93 Patentblatt 93/39**

(84) Benannte Vertragsstaaten : **CH DE FR GB LI**

(56) Entgegenhaltungen :
DE-A- 1 467 437
DE-A- 2 403 211
DE-A- 2 446 546
DE-B- 2 403 211
DE-B- 2 405 578
DE-C- 2 403 211
Chemical Abstracts, Band 83, Nr. 16, 20. Oktober 1975 (Columbus, Ohio, US), siehe Seite 355, Spalte 1, Zusammenfassung 136949n, JP, A, 7548021, 28. April 1975, Showa Pharmaceutical Chemical Industry Co.
Research Disclosure, Nr. 162, Oktober 1977 (GB), "New Dental Materials", siehe Seite 80, Spalte 1, Zusammenfassung 16269
Reports of the Institute for med. and dent. eng. 1968, 55-61 (Hirasawa et al.)

(56) Entgegenhaltungen :
Sonderdruck Nr. 32 der Fa. Degussa auch Chemie, Ingenieur Technik 48, II (1976), Chemie in unserer Zeit 13, 1979, Nr. 5, pp. 142-146
Chemie in unserer Zeit, 13. Jg. 1979, Nr. 5, S. 142-146
Römpps Chemie Lexikon, 7. Auflage, Seite 1769
Merkblatt "Silton" Mizusawa Kagaku Kogyo
Römpps Chemie Lexikon, 8. Auflage, Seite 2106
Technisches Bulletin Ivoclar/Vivadent, Februar 1980

(73) Patentinhaber : ESPE Stiftung & Co Produktions- und Vertriebs KG
Am Griesberg 2
D-82229 Seefeld (DE)

(72) Erfinder : SCHMITT, Werner
Prinzenweg 10
D-8130 Starnberg (DE)
Erfinder : PURRMANN, Robert
Riemerschmidstrasse 18
D-8130 Starnberg (DE)
Erfinder : JOCHUM, Peter
Pointweg 5
D-8031 Hechendorf (DE)
Erfinder : HÜBNER, Heinz-Joachim
Moosbichelweg 16
D-8031 Wörthsee (DE)

(74) Vertreter : Hepp, Dieter et al
HEPP & Partner AG Marktgasse 18
CH-9500 Wil (CH)

EP 0 040 232 B2

**Beschreibung**

Die Erfindung betrifft die Verwendung von silanisierter Kieselsäure als Füllstoff in polymerisierbaren Dentalmassen auf Basis äthylenisch ungesättigter Monomerer ausgewählt aus di- oder polyfunktionellen Derivaten der Acryl- oder Methacrylsäure.

Den härtbaren Dentalmassen auf Basis äthylenisch ungesättigter, polymerisierbarer Monomerer, von denen besonders die mono- und di-bzw. polyfunktionellen Ester der Acryl- und Methacrylsäure grosse praktische Anwendung gefunden haben, werden zur Verringerung des Polymerisationsschrumpfes, zur Erniedrigung des thermischen Expansionskoeffizienten und zur Erhöhung der Härte der erhaltenen Polymerisate grössere Mengen an anorganischen Füllstoffen zugesetzt, wie aus der US-Patentschrift Nr. 3066112 bekannt ist. Der Anteil an inerten anorganischen Füllstoffen kann in derartigen dentalen Dauerfüllungsmassen bis zu über 80% der Gesamtmasse betragen. Als Füllstoffe werden z.B. Quarz, Quarzglas oder Silikatgläser, wie Lithiumaluminiumsilikat oder Bariumsilikatglas als feine Pulver verwendet. Die Korngrössen dieser Füllstoffe liegen im Bereich von 1 bis ca. 100 µm, wobei im allgemeinen der mittlere Teilchendurchmesser in der Grössenordnung von etwa 10 µm liegt.

Nachteilig an der Verwendung dieser bekannten Füllstoffe ist die noch nicht befriedigende Abriebfestigkeit der daraus hergestellten Dentalmaterialien sowie ihre Oberflächenrauhigkeit beim Einsatz als Zahnfüllungen. Durch ihr mässiges Abriebverhalten konnten die Zahnfüllmaterialien mit den genannten Füllstoffen nicht im Molarengebiet eingesetzt werden, wo trotz des toxikologisch bedenklichen Quecksilbers die Amalgamfüllungen noch vorherrschen. Die Oberflächenrauhigkeit der genannten Materialien führt auch bei der Verwendung im Frontzahngebiet zu Problemen, da hierdurch die Ablagerung von Zahnbelag gefördert wird und dadurch sowohl Verfärbungen als auch marginale Sekundärkaries verursacht werden können.

Aus der DE-OS Nr. 2403211 ist es bekannt, dass sehr feinteilige Füllstoffe, wie mikrofeine Kieselsäure (Siliciumdioxidgel) oder Aluminiumoxid, mit Teilchengrössen im Bereich von 5-700 nm nach dem Aushärten der damit hergestellten Dentalmassen gut polierbare Formkörper ergeben. Wie aus den Artikeln von R. Schäfer im "Dental Echo", Bd. 49, 136 (1979) und A. Gross in "Chemie in unserer Zeit", 13. Jahrgang, S. 142 (1979) zu entnehmen ist, können jedoch mit diesen mikrofeinen Füllstoffen, die eine sehr grosse spezifische Oberfläche besitzen, allein keine formbaren Pasten mit den hohen Füllstoffgehalten erhalten werden, wie sie für die dentale Verwendung in der zahnärztlichen Praxis erforderlich sind. Es zeigt sich nämlich, dass beim Einmischen der mikrofeinen Kieselsäure, z.B. des pyrolytisch hergestellten Siliciumdioxidgels, in das flüssige Monomer, dieses sich zunächst zu einer honigartigen, fadenziehenden, klebrigen Masse verdickt, um dann bei fortgesetzter Zugaben der Kieselsäure relativ plötzlich in eine bröckelige Masse überzugehen, die sich schliesslich in ein frei fliessendes Pulver auflöst. Daher können hiermit nur Massen mit salbenartiger oder pulveriger Konsistenz hergestellt werden. In diesen Literaturstellen wird nun angegeben, dass der mikrofeine Füllstoff zunächst mit einem polymerisierbaren Monomer angeteigt und vorpolymerisiert werden soll. Das so erhaltene feste Produkt wird zerkleinert und fein gemahlen. Dieses feinteilige Splitterpolymerisat wird dann als eigentlicher Füllstoff in den härtbaren Dentalmassen verwendet.

Auf diese Weise können jedoch keine sehr hohen Anteile an anorganischem Füllstoff in den Dentalmassen realisiert werden, da nach diesem bekannten Verfahren das in das Monomer einzuarbeitende Splitterpolymerisat bereits selbst zu einem grossen Anteil aus organischer Substanz besteht. Bei Verwendung dieses Füllstoffes ist daher ein höherer thermischer Expansionskoeffizient in Kauf zu nehmen, wodurch der erstrebte Vorteil des hohen Füllstoffanteils in den polymerisierbaren Dentalmassen teilweise wieder aufgehoben wird. Wenn Füllungsmaterial und Zahnsubstanz einen stark unterschiedlichen thermischen Expansionskoeffizienten aufweisen, wird durch den z.B. die Nahrungsaufnahme bedingten Temperaturwechsel der sogenannte "Pumpeffekt" hervorgerufen. Durch den gebildeten Randspalt können Bakterien eindringen und zu Sekundärkaries oder in tiefen Kavitäten zu Pulpenschädigungen führen. Auch muss bei der Herstellung des Vorpolymerisats die mikrofeine Kieselsäure mit der Monomerflüssigkeit sehr intensiv gemischt werden, damit ein gleichmässiges Überziehen der Teilchen des Siliciumdioxidpulvers mit dem härtbaren Monomer erreicht wird. Die kalthärtenden Monomerzubereitungen sind wegen ihres relativ raschen Polymerisationsverlaufs hierfür kaum geeignet. Man muss daher auf das Verfahren der Heisspolymerisation zurückgreifen. Die dabei auftretenden relativ hohen Temperaturen führen jedoch zu Verfärbungen, zumal die Aushärtung möglichst intensiv erfolgen soll, damit bei diesem Vorpolymerisieren ein hartes, sprödes, leicht pulverisierbares Füllstoffmaterial erhalten wird. Wegen dieser mehr oder weniger stark gelblich gefärbten Vorpolymerisatpulver ist es schwierig, mit ihnen Dentalmassen in heller Farbgebung zu erhalten und bei der technischen Herstellung eine gleichbleibende Farbeinstellung zu gewährleisten. Zwar hat man auch bereits die mikrofeine, pyrogene Kieselsäure mit Silanisierungsmitteln behandelt, um die Haftung des anorganischen Füllstoffes mit dem Polymer zu verbessern. Hierbei tritt auch eine gewisse Agglomerierung der Pulverteilchen ein, doch besitzen diese Füllstoffe nur eine sehr geringe Härte, da diese Agglomerate nicht hart und beständig sind, weil die Bindung durch die Silanisierungsmittel nicht stabil ist. Beim Einarbeiten dieser Agglomerate in die Monomerzubereitung tritt eine erneute Zerteilung ein und es werden wiederum nur salben- oder pulverartige Produkte erhalten.

Relativ stabil agglomerierte Kieselsäuren sind aus der DE-OS Nr. 1467437 bekannt; ihre vorteilhafte Verwendung für die speziellen Anforderungen von Dentalmassen geht daraus jedoch in keiner Weise hervor, denn dort wird die Verwendung als Mattierungsmittel für Lacke beschrieben.

Aus dem Aufsatz von Hirasawa et al in "Reports of the Institute for Medical and Dental Engineering", 1968, Seite 55 bis 61, ist die Verwendung einer nur durch Dichte und Primärteilchengrösse grob charakterisierten Kieselsäure als Füllstoff von Polymerisaten aus monomerem Methylmethacrylat bekannt. (Gemäss den angegebenen Werten dürfte es sich um Fällungskieselsäure handeln.) Hirasawa beschreibt weder die Einhaltung bestimmter Sekundär-Teilchengrössen noch wird auf eine Granulierung oder anderweitige Vorbehandlung der Primärteilchen oder eine Verwendung/Mitverwendung pyrogener Kieselsäure eingegangen. Aus der Veröffentlichung ergeben sich wesentliche Nachteile, wie insbesondere Sedimentationsneigung, Quellprobleme, sowie ein in der Praxis nicht anwendbares Polymerisationsverfahren.

Aus der DE-B-24 05 578 ist die Herstellung eines Dentalwerkstoffs unter Verwendung amorpher Kieselsäure bekannt. Als amorphe Kieselsäure wird dabei gefällte oder durch Flammhydrolyse hergestellte Kieselsäure verstanden. Die Entgegenhaltung differenziert dabei nicht zwischen Primär-Teilchengrösse und Sekundär-Teilchengrösse. Auch wird nicht die durchschnittliche Teilchengrösse des Füllstoffs angegeben.

In der Praxis hat sich gezeigt, dass sich mit solchen Füllstoffen keine zuverlässigen Rezepturen für formbare Dentalmassen erhalten lassen, wie sie zur Verarbeitung in der zahnärztlichen Praxis erforderlich sind. Vielmehr sind unerwünscht niedrige Füllstoffanteile, das unberechenbare Mischungsverhalten, wie vorstehend im Zusammenhang mit der DE-A-24 03 211 aufgezeigt, und die Notwendigkeit des Umwegs über Splitterpolymersiat-Herstellung die Folge.

Aus der DE-A-24 46 546 ist ein Verfahren zur Herstellung eines Füllstoffs auf der Basis natürlichen Siliciumdioxyds bekannt. Zweck dieses bekannten Verfahrens ist es, Grauverfärbungen zu vermeiden, die normalerweise bei Verwendung natürlicher Mineralstoffe auftreten. Der Füllstoff wird einer Säure- und Temperaturbehandlung unterworfen, ohne dass dabei eine Veränderung der Teilchengrösse beabsichtigt oder bwirkt würde.

Aufgabe der Erfindung ist es, die Nachteile des Bekannten zu vermeiden, insbesondere also die Herstellung von Dentalmassen zu ermöglichen, die höheren Füllstoffgehalt aufweisen, gut verarbeitet werden können, verbesserte Abriebfestigkeit und thermische Eigenschaften (kein "Pumpeffekt") aufweisen und im übrigen Dentalmassen mit füllstoffhaltigen Splitterpolymerisation auch hinsichtlich Polierbarkeit und mechanischer und optischer Eigenschaften wenigstens gleichwertig sind.

Erfindungsgemäss wird dies bei Verwendung der eingangs genannten Kieselsäure in den polymerisierbaren Dentalmassen dadurch erreicht, dass

a) ein aus pyrogener Kieselsäure mit oder ohne Bindemittel hergestelltes anorganisches Granulat mit einer mittleren Korngrösse des Granulats von 0,5 bis 50 μm und einer mittleren Primärteilchengrösse von 1 bis 100nm, und ggf. weitere anorganische Bestandteile enthaltend, sowie

b) eine nicht granulierte, mikrofeine Rieselsäure einer mittleren Teilchengrösse von 1 bis 100 nm,

c) mit Gewichtsanteilen von (a) und (b), bezogen auf die Gesamtmasse, in Höhe von 50% bis 80% und von (b) in Höhe von 2% bis 30% verwendet werden, und wobei

d) das Kieselsäuregranulat (a) nach der Verarbeitung mit den Monomeren unzerteilt in der mittleren Korngrösse von 0,5 bis 50 μm vorliegt.

Die Granulate müssen einerseits so beständig sein, dass sie beim Verarbeiten mit den Monomeren nicht wieder zerteilt werden und dürfen andererseits nur so hart sein, dass die Formkörper nach dem Polymerisieren nach polierbar sind. Die Härte der Füllstoffe kann im Rahmen der anschliessend beschriebenen Herstellungsverfahren durch die Dauer und besonders die Temperatur der abschliessenden Glühung eingestellt werden; der geeignete Temperaturbereich beträgt dabei von ca. 600 bis über 1200°C. Naturgemäss muss die Temperatur und Glühdauer der jeweiligen Kombination Kieselgel/oxidisches Bindemittel angepasst werden, um die jeweils gewünschten Härtewerte zu erreichen, was der Fachmann durch einfache Versuche ermitteln kann.

Die Kieselsäuregranulate können auf verschiedenem Wege mit oder ohne Bindemittel hergestellt werden. Für eine Bindung der Primärteilchen mit Siliciumdioxid eignet sich ein Verfahren, bei dem die mikrofeine Kieselsäure, die bei dem die mikrofeine Kieselsäure, die auf pyrogenem Wege erhalten wurde und deren Teilchengrösse von 1 bis 100 nm, vorzugsweise von 5 bis 50 nm betragen kann, mit einer Wasserglaslösung angeteigt wird, worauf die Mischung angesäuert, die Masse getrocknet und langsam auf über 600° C erhitzt wird. Dann wird mit Wasser gewaschen, wodurch lösliche Anteile entfernt werden. Nach dem Trocknen wird das so erhaltene Granulat gegebenenfalls durch Mahlen und Sieben auf die gewünschte Korngrösse eingestellt.

In ähnlicher Weise lässt sich auch durch Verwendung von wässeriger Borsäure- oder Alkaliboratlösung, wässeriger Aluminatlösung oder alkoholischer Aluminiumalkoholatlösung eine Bindung und stabile

EP 0 040 232 B2

Agglomerierung zu einem anorganischen Kieselsäuregranulat erreichen, das als Füllstoff geeignet ist. Während die Aluminiumverbindungen die Granulierung wohl durch die Bildung des Bindemittels Aluminiumoxid bewirken, haben die Borverbindungen anscheinend überwiegend katalytische Wirkung für die Bindung der Primärteilchen im Granulat, die Hauptmenge des gebildeten Boroxids wird beim Glühschritt verflüchtigt ohne die Stabilität des Granulats zu beeinflussen. Zwar war es aus der DE-OS Nr. 2716225 bekannt, dass man Fällungskieselsäure mit Borsäure imprägnieren kann. Die Kieselsäure dient hierbei als Katalysatorträger für das Boroxid und als Füllstoff für Organopolysiloxane, die als sogenannte Springkitte verwendet werden. Daher konnte dieser Stand der Technik keine Anregung für die vorteilhafte Verwendung dieser borsäuregebundenen Kieselsäuregranulate für die speziellen Anforderungen von Dentalmassen geben.

Gemäss einem anderen geeigneten Herstellungsverfahren wird das Ausgangsmaterial mit Siliciumtetrachlorid angeteigt und dann in feuchter Atmosphäre oder durch Zugabe von Wasser hydrolysiert. Die gebildete Mischung wird langsam auf über 600° C erhitzt, wobei die gebildete Salzsäure entweicht. Anschliessend wird HCl-frei gewaschen und durch Mahlen und Sieben auf die gewünschte Korngrösse eingestellt.

Mann kann diese Verfahren auch kombinieren in der Weise, dass der Mischung aus Ausgangskieselsäure und Wasserglas das Siliciumtetrachlorid zugefügt wird. Die weitere Verarbeitung erfolgt dann wie beschrieben durch Trocknen und Erhitzen und nachfolgendes Auswaschen löslicher Bestandteile wie z.B. Alkalichlorid.

Nach einer weiteren, vorteilhaften Herstellungsweise wird die mikrofeine Kieselsäure mit Organosiliciumverbindungen, die vorzugsweise einen polymerisierbaren Rest enthalten, angeteigt. Gegebenenfalls wird ein Polymerisationskatalysator in üblichen Konzentrationen zugefügt und die Mischung z.B. in der Hitze polymerisiert. Anschliessend wird das Gemisch langsam an Luft erhitzt, bis die organischen Bestandteile verbrannt sind und schliesslich auf über 600' C gebracht. Nach dem Abkühlen kann durch Mahlen und Sieben die gewünschte Korngrösse erhalten werden.

Geeignete Organosiliciumverbindungen sind z.B.: Vinyltrichlorsilan, Vinyltrimethoxysilan, Allyldimethylchlorsilan, $\gamma$-Methacryloxypropyltrimethoxysilan, $\beta$-(3,4-Epoxycyclohexyl)äthyltrimethoxysilan und $\gamma$-Glycidoxypropyltrimethoxysilan. Auch längeres Glühen der mikrofeinen Kieselsäuren oberhalb 800° C ohne bindende Zusätze führt zu brauchbaren Granulaten.

Das erfindungsgemässe Kieselsäuregranulat lässt sich gut mit den flüssigen polymerisierbaren Monomeren vermischen. Dabei haben sich als Monomere die mono-, die- oder polyfunktionellen Derivate der Acryloder Methacrylsäure bewährt, insbesondere die Ester. In vorteilhafter Weise wird der erfindungsgemässe Füllstoff zur Einstellung der Konsistenz und zur Verhinderung des Absetzens, zusammen mit einem Anteil an nicht granulierter, mikrofeiner Kieselsäure einer Teilchengrösse von 1 bis 100 nm, vorzugsweise von 5 bis 50 nm, verwendet.

Durch die Verwendung von aus pyrogener Kieselsäure hergestellten Granulaten lässt sich eine Dentalmasse industriell herstellen, die Füllstoff-Gewichtsanteile, bezogen auf die Gesamtmasse, in Höhe von 50% bis 80% ermöglicht.

Das Kieselgelgranulat kann vor seiner Verwendung auch silanisiert werden, um die Bindung mit dem Polymer zu verbessern, z.B. durch Behandlung mit Trimethoxy-(3-methacryloxypropyl)silan. Bei Verwendung in Dentalpräparaten wird in der Regel zur Anpassung an die natürlichen Zähne die Masse noch mit bekannten organischen oder anorganischen Farbpigmenten und/oder Trübungsmitteln versetzt werden. Als Härtungskatalysatoren können in diesen Präparaten u.a. organische Peroxide, wie Dibenzoylperoxid oder Azoverbindungen, wie Azobisisobutyronitril eingesetzt werden. Auch die für die Kalthärtung von vinylungesättigten Monomeren geeigneten Redoxsysteme, wie z.B. Dibenzoylperoxid/N,N-Bis-2-hydroxyäthylxylidin oder Dibenzoylperoxid/Barbitursäure-Derivate sind geeignet. Als Härtungskatalysatoren können aber auch Substanzen eingesetzt werden, die nach Bestrahlung mit UV- oder sichtbarem Licht die Polymerisation auslösen, wie z.B. Benzoinalkyläther, Benzilmonoketale oder aliphatische und aromatische 1,2-Diketonverbindungen, wobei die Lichtpolymerisation durch Zusätze von Aktivatoren, wie Aminen oder organischen Phosphiten, in an sich bekannter Weise beschleunigt werden kann.

Nach dem Aushärten der polymerisierbaren Massen mit den erfindungsgemässen Füllstoffen ist die Druckfestigkeit der Formkörper um bis zu mehr als 20% höher als bei Verwendung der Füllstoffe gemäss dem Stand der Technik. Sie beträgt bei den Photopolymerisaten statt etwa 350 bis über 450 MPa. Ausserdem werden die guten Eigenschaften der quarzgefüllten Formkörper, nämlich geringe thermische Expansion, hohe Farbstabilität, geringe Wasseraufnahme, mit guten Eigenschaften der vorbekannten mit mikrofeiner Kieselsäure hergestellten Formkörper hinsichtlich Polierbarkeit in einem Material vereinigt, ohne dass hierbei Nachteile in Kauf genommen werden müssen. Es war nicht zu erwarten, dass durch die Verwendung mechanisch relativ stabiler Agglomerate aus mikrofeiner, pyrogener Kieselsäure und gegebenenfalls rein anorganischem Bindemittel als Füllstoff zusammen mit polymerisierbaren Monomeren pastöse Dentalmassen mit guter Konsistenz trotz hohem Füllstoffanteil erhalten werden können, die nach dem Aushärten hochglanzpolierbar sind.

Die erfindungsgemässen Füllstoffgranulate eignen sich allgemein für Zahnrestaurationsmassen, also

4

nicht nur für Zahnfüllpräparate, sondern auch für solche Zwecke, für die derartige Werkstoffe üblicherweise vorteilhaft verwendet werden, z.B. zur Herstellung von Kronen, Brücken, Verblendungen und ähnlichen Zahnersatzteilen oder auch zur Herstellung von künstlichen Zähnen.

Insgesamt vereinigt die Erfindung eine Vielzahl bisher scheinbar unvereinbarer Vorteile und positiver Materialeigenschaften auf sich. Namentlich führt die erfindungsgemässe Verwendung zu hohem Füllstoffgehalt und trotzdem hervorragender Verarbeitbarkeit (kein Fadenziehen und kein Bröckeln). Die Granulat-Korngrösse liegt im Mikrobereich und trotzdem ergeben sich gute Polierbarkeit, gute optische Eigenschaften und eine verbesserte Farbstabilität. Besonders durch die Erhöhung der Füllstoffanteile wird die Druckfestigkeit verbessert, ohne dass dabei schlechtere Polierbarkeit in Kauf genommen werden müsste. Auch die Biegefestigkeit wird dadurch erhöht und trotzdem noch die thermische Ausdehnung, der negative "Pumpeffekt", verringert.

*Beispiel 1*

*Granulierung mit Wasserglas*

In 100 g filtriertes Natronwasserglas (ca. 39° Bé) wird die unten angegebene Menge Kieselgel eingeknetet und die Mischung mit conc. Salzsäure auf pH 5 bis 6 eingestellt. Durch Zugabe von Methanol wird eine knetbare Konsistenz erhalten. Nach 2stündigem Kneten wird bei 60°C und 200 Torr das Methanol entfernt und durch Erwärmen auf 120°C getrocknet. Anschliessend wird langsam auf 800°C gebracht. das erkaltete Produkt gemahlen und durch Waschen mit Wasser vom löslichen Alkaligehalt befreit. Durch erneutes Trocknen bei 120°C wird ein feinpulveriger Füllstoff erhalten (mittlere Korngrösse ca. 5 $\mu$m).

| Produkt Nr. | Ausgangskieselgel | | | Menge (g) |
|---|---|---|---|---|
| | Herstellungsart | spez. Oberfläche ($m^2/g$) | mittl. Teilchendurchmesser (nm) | |
| 1.1 | pyrogen | 50 | 40 | 60 |
| 1.2 | pyrogen | 380 | 7 | 25 |
| **1.3** | **gefällt** | **110** | **28** | **60** |

*Beispiel 2*

*Granulierung mit Borsäure*

Bei ca. 78°C wird durch Dekantieren vom Bodensatz eine gesättigte, wässerige Borsäurelösung hergestellt. In 468 g dieser Lösung werden bei einer Temperatur von über 80°C während ca. 2 h 1400g pyrogene Kieselsäure (spezifische Oberfläche 50 m2/g, mittlerer Teilchendurchmesser 40 nm) unter Rühren eingetragen. Anschliessend wird 30 min weitergerührt und dann das Wasser im Vakuum entfernt. Der Feststoff wird bei 120°C getrocknet und langsam weiter auf 800°C erhitzt. Nach Mahlen in der Kugelmühle wird das erhaltene Pulver mit Wasser extrahiert bis das Waschwasser borfrei ist. Anschliessend wird der Füllstoff im Vakuum bei 120°C getrocknet.
Die mittlere Korngrösse des Füllstoffes beträgt ca. 4$\mu$m.

*Beispiel 3*

*Paste/Paste-Präparat zur Herstellung von Zahnfüllungen*

Zur Herstellung der Pasten werden verknetet:
A) 249,0 g eines zahnähnlich eingefärbten Kieselsäuregranulats (mittlere Teilchengrösse 5 $\mu$m, mittlere Primärteilchengrösse 40 nm, Bindemittel $SiO_2$, < 63 $\mu$m, silanisiert), und
33,0 g pyrogenes, nicht granuliertes, silanisiertes Kieselgel
mit einer Lösung von
1,5g N,N-Bishydroxyäthyl-3,5-di-t-butylanilin in
122,5 g 2,2-Bis[p-($\gamma$-hydroxypropoxy)phenyl]propandimethacrylat, und

31.0 g Bis-GMA

B) 287,0 g des in Paste A verwendeten, silanisierten Kieselsäuregranulats, und

42,0 g pyrogenes, nicht granuliertes, silanisiertes Kieselgel

mit einer Lösung von

4,0 g p-Chlorbenzoylperoxid in

200,0 g 2,2-Bis[p-($\gamma$-hydroxypropoxy)phenyl]propandimethacrylat, und

40,0 g Bis-GMA

Durch Vermischen gleicher Teile der beiden Pasten entsteht eine plastische Masse, die gut in üblich präparierte Zahnkavitäten eingebracht und modelliert werden kann. Ca. 2 min nach dem Anmischen beginnt die Erhärtung, die nach ca. 3,5 min beendet ist. Die abriebfeste Füllung kann durch übliche zahnärztliche Massnahmen hochglanzpoliert werden. Der lineare thermische Expansionskoeffizient des ausgehärteten Materials beträgt $45 \cdot 10^{-6} K^{-1}$.

*Beispiel 4*

*Durch UV-Licht polymerisierbares Zahnfullmaterial*

Eine Lösung wird hergestellt aus:

20,0 g Bishydroxymethyltricyclo-[5.2.1.0,$^{2,6}$]decandimethacrylat (stabilisiert mit 200 ppm p-Methoxyphenol und 200 ppm Jonol)

120,0 mg Benzildimethylketal, und

100,0 mg Didecylphenylphosphit

Weiterhin bereitet man ein Pulvergemisch aus

14,0 g silanisiertem, erfindungsgemässen Kieselsäuregranulat (mittlere Teilchengrösse 6 $\mu$m, mittlere Primärteilchengrösse 40 nm, Bindemittel $B_2O_3$, < 63 $\mu$m), zahnähnlich eingefärbt

10,0 g silanisiertem, nicht granulierten, pyrogenen Kieselgel, und

2,0 g feinteiligem Calciumfluorid.

10,5 g der Lösung und 18 g des Pulvergemischs werden zu einer homogenen Zahnfüllmasse verknetet. Nach einer Belichtungszeit von 20 s mit einem handelsüblichen UV-Bestrahlungsgerät (Uviolite, Firma ESPE) mit 70 mW Lichtleistung härtet das Material in einer Schichtdicke von ca. 3 mm aus. Das abrasionsresistente Material ist hochglanzpolierbar und hat einen linearen thermischen Expansionskoeffizienten von $45 \cdot 10^{-6} K^{-1}$.

Die Druckfestigkeit, gemessen an belichteten Prüfkörpern von $2 \times 2 \times 4$ mm nach 24stündiger Lagerung unter Wasser bei 36°C beträgt 400 MPa.

*Vergleichsversuch 1*

In ein Gemisch aus

14,0 g Bishydroxymethyltricyclo-[5.2.1.0$^{2,6}$]decandiacrylat (stabilisiert mit 200 ppm p-Methoxyphenol und 200 ppm Jonol)

6.0 g Bis-GMA

9,0 g silanisierter, pyrogener Kieselsäure (spezifische Oberfläche 50 m$^2$/g)

0,3 g Methyldiäthanolamindimethacrylat, und

0,03 g Campherchinon

wird zum einen von handelsüblichen Kieselsäuren und zum anderen von erfindungsgemässen Granulaten auf Basis dieser Kieselsäuren eine durch Vorversuche bestimmte Menge eingebracht, die gerade noch eine pastöse Masse ergibt. Die nach Belichten mit einem handelsüblichen, sichtbares Licht emittierenden dentalen Belichtungsgerät (Elipar-Gerät, Firma ESPE) erhaltenen Prüfkörper zeigen die in der folgenden Tabelle aufgeführten physikalischen Eigenschaften.

| Typ | Kieselsäure mittl. Teilchen größe [nm] | granu- liert | Verarbeitbar als Zahnfüll- Material | Ober- flächen Härte [MPa] | Thermische Expansion [K⁻¹] | Biege- festigkeit [MPa] | Anorg. Anteil [% der Gesamt- masse] | Bemerkung |
|---|---|---|---|---|---|---|---|---|
| pyrogen | 40 | nein | nein (fadenzieh.) | 128 | $62 \cdot 10^{-6}$ | 65 | 58 | Handelspro- dukt Aerosil OX50 Firma Degussa |
| pyrogen | 40 | ja | gut | 202 | $52 \cdot 10^{-6}$ | 85 | 60 | |
| pyrogen | 7 | nein | nein (fadenzieh.) | 160 | $78 \cdot 10^{-6}$ | 72 | 32 | Handelspro- dukt Aerosil 380 Fir- ma Degussa |
| pyrogen | 7 | ja | gut | 255 | $46 \cdot 10^{-6}$ | 93 | 68 | |

Nur die Verwendung der erfindungsgemäss granulierten Kieselsäuren liefert Pasten, die als Zahnfüllmasse verarbeitet werden können. Oberflächenhärte der Polymerisate steigt um mehr als 50%, die thermische Expansion verringert sich um 15 bis 50%, die Biegefestigkeit erhöht sich um mehr als 30%.

*Vergleichsversuch 2*

Eine erfindungsgemässe Zahnfüllmasse A gemäss Beispiel 3 wird in ihren physikalischen Daten verglichen mit den handelsüblichen Präparaten B, C und D, deren Basis folgende Füllkörper darstellen:

B) Glaspulver, mittlere Korngrösse ca. 10 µm, (Adaptic Radiopaque, Firma Johnson & Johnson)

C) Glaspulver, mittlere Korngrösse ca. 10 µm gemischt mit mikrofeiner Kieselsäure (Miradapt, Firma Johnson & Johnson)

D) Einpolymerisierte, mikrofeine Kieselsäure, mittlere Teilchengrösse ca. 40 nm (Silar, Firma 3M Co.)

| | Präparat A | B | C | D |
|---|---|---|---|---|
| Druckfestigkeit [MPa] | 370 | 240 | 320 | 290 |
| Biegefestigkeit [MPa] | 80 | 65 | 85 | 50 |
| Farbstabilität | hervorr. | gut | gut | mäßig |
| Polierbarkeit | hervorr. | schlecht | mäßig* | hervorr. |
| (* = nur mit speziellen Schleifinstrumenten) | | | | |

Die erfindungsgemässe Zahnfüllmasse A vereinigt in sich die Vorteilhaften Eigenschaften der herkömmlichen Massen mit den Glasfüllkörpern (Präparat B) sowie mit den einpolymerisierten mikrofeinen Kieselsäuren (Präparat D), was dem blossen Gemisch aus beiden Füllkörperarten (Präparat C) nicht gelingt.

Nur die erfindungsgemässe Masse ist polierbar bei hoher Druck- und Biegefestigkeit und hervorragender Farbstabilität.

## Patentansprüche

1. Verwendung von silanisierter Kieselsäure als Füllstoff in polymerisierbaren Dentalmassen auf Basis äthylenisch ungesättigter Monomerer ausgewählt aus di- oder polyfunktionellen Derivaten der Acryl- oder Methacrylsäure, wobei

(a) ein aus pyrogener Kieselsäure mit oder ohne Bindemittel hergestelltes anorganisches Granulat mit einer mittleren Korngrösse des Granulats von 0,5 bis 50 µm und einer mittleren Primärteilchengrösse von 1 bis 100 nm, und ggf. weitere anorganische Bestandteile enthaltend, sowie

7

(b) eine nicht granulierte, mikrofeine Kieselsäure einer mittleren Teilchengrösse von 1 bis 100 nm,

(c) mit Gewichtsanteilen von (a) und (b), bezogen auf die Gesamtmasse, in Höhe von 50% bis 80% und von (b) in Höhe von 2% bis 30% verwendet werden, und wobei

(d) das Kieselsäuregranulat (a) nach der Verarbeitung mit den Monomeren unzerteilt in der mittleren Korngrösse von 0,5 bis 50 μm vorliegt.

2. Verwendung nach Anspruch 1, wobei die Gewichtsanteile von (a) und (b) 50% bis 70%, bezogen auf die Gesamtmasse, betragen.

3. Verwendung von Kieselsäuregranulaten nach Anspruch 1, die durch Glühen zwischen 600° und über 1200°C erzeugt sind.

4. Verwendung nach Anspruch 3, wobei die Kieselsäuregranulate durch Glühen in Anwesenheit von Bindemittel erzeugt sind.

5. Verwendung von Kieselsäuregranulaten nach einem der vorangehenden Ansprüche mit einer mittleren Primärteilchengrösse von 5 bis 50 nm.

## Claims

1. Use of silanised silicic acid as filler in polymerizable dental masses on the basis of ethylenically unsaturated monomers, in particular from difunctional or polyfunctional derivatives of acrylic acid or methacrylic acid, whereby

a) an anorganic granulate, manufactured from pyrogenic silicic acid with or without binders, with an average grain size of the granulate of between 0,5 and 50 um and an average primary particle size of between 1 and 100 nm, and optionally containing further anorganic components, as well as

b) a non-granulated, microfine silicic acid with an average grain size of between 1 and 100 nm,

c) with weight proportions of (a) and (b) being used amounting to between 50% and 80% in relation to the total weight, and of (b) amounting to between 2% and 30%, and whereby

d) the silicic acid granulate (a) is available which has not disintegrated, with an average grain size of between 0,5 and 50 um, after processing with the monomers.

2. Use according to claim 1, the weight proportions of (a) and (b), in relation to the total weight, amounting to between 50% and 70%.

3. Use of silicic acid granules according to claim 1, which are created through heating between 600 C and above 1200 C.

4. Use according to claim 3, the silicic acid granules being created by heating in the presence of binders.

5. Use of silicic acid granules according to one of the preceding claims with an average primary particle size of between 5 and 50 nm.

## Revendications

1. Utilisation d'une silice silanisée en tant que matière de charge dans des masses dentaires polymérisables à base de monomères à insaturation éthylénique choisis parmi les dérivés di- ou poly-fonctionnels de l'acide acrylique ou méthacrylique, dans laquelle on utilise :

a) des granules minéraux préparés à partir d'une silice de pyrogénation avec ou sans liant, à une dimension moyenne des granules de 0,5 à 50 μm et une dimension de particule primaire moyenne de 1 à 100 nm, et contenant le cas échéant d'autres constituants minéraux, et

b) une silice microfine non granulée, à une dimension de particule moyenne de 1 à 100 nm,

c) avec des proportions en poids de (a) et (b), par rapport à la masse totale, à un niveau de 50 à 80 % et des proportions relatives de (b) à un niveau de 2 à 30 %,

d) les granules de silice (a), non fragmentés après le mélange avec les monomères, ayant une dimension de grain moyenne de 0,5 à 50 μm.

2. Utilisation selon revendication 1, dans laquelle les proportions en poids de (a) et (b) sont de 50 à 70 % par rapport à la masse totale.

3. Utilisation de granules de silice selon revendication 1, qui ont été obtenus par calcination entre 600°C et plus de 1200°C.

4. Utilisation selon revendication 3, dans laquelle les granules de silice ont été obtenus par calcination en présence de liants.

5. Utilisation de granules de silice selon une des revendications qui précèdent, à une dimension de particule primaire moyenne de 5 à 50 nm.